# EUROPÄISCHE PATENTSCHRIFT

(11) **EP 0 429 998 B1**
(45) Veröffentlichungstag und Bekanntmachung des Hinweises auf die Patenterteilung: **05.01.1994**
(21) Anmeldenummer: 90121994.9
(22) Anmeldetag: 17.11.1990
(51) Int. Cl.: C07C 29/00, C07C 35/48, C07C 31/38, C07C 41/16, C07C 43/17

(54) **Verfahren zur Herstellung teilfluorierter Alkohole**
Process for the preparation of partially fluorinated alcohols
Procédé pour la préparation d'alcools partiellement fluorés

(30) Priorität: 30.11.1989 DE 3939535
(43) Veröffentlichungstag der Anmeldung: 05.06.1991
(73) Patentinhaber: BAYER AG, 51368 Leverkusen (DE)
(72) Erfinder: Gassen, Karl-Rudolf, Dr., W-5068 Odenthal (DE); Bielefeldt, Dietmar, Dr., W-4030 Ratingen 6 (DE); Negele, Michael, Dr., W-5000 Köln 80 (DE); Ziemann, Heinz, Dr., W-5653 Leichlingen (DE)

(56) Entgegenhaltungen:
- EP-A- 0 157 739
- EP-A- 0 364 587
- DE-B- 1 244 169
- US-A- 2 559 628

## Beschreibung

Die vorliegende Erfindung betrifft ein vorteilhaftes Verfahren zur Herstellung von teilfluorierten Alkoholen ausgehend von halogenhaltigen Alkenen sowie 2,2,3,3,4,4-Hexafluorcyclopentanol als neuer teilfluorierter Alkohol.

Fluorhaltige Alkohole sind wertvolle Zwischenprodukte für die Synthese von Wirkstoffen, die in der Pharmazie, in der Landwirtschaft oder als Farbstoffe eingesetzt werden können (vgl. z.B. J.T. Welch, Tetrahedron (1987) 3123). Im Bereich der Landwirtschaft eignen sich die teilfluorierten Alkohole insbesondere als Zwischenprodukte zur Herstellung von Schädlingsbekämpfungsmitteln.

Ferner besitzen fluorhaltige Alkohole gute Lösemittel-Eigenschaften (vgl. H. Liebig und K. Ulm, Chemiker Zeitung 12, 477 (1975)).

Die bisher bekannten Verfahren erlauben nur die Darstellung einiger ausgewählter Vertreter der Klasse der teilfluorierten Alkohole. Ein Verfahren beinhaltet die Umsetzung von Tetrafluorethen mit Vinylacetat unter Druck. Durch anschließende Verseifung gelangt man so zum 2,2,3,3-Tetrafluorcyclobutanol (W.D. Phillips, J. Chem. Phys. 25 (1956) 949). Nachteilig an diesem Verfahren ist, daß es nicht im technischen Maßstab durchführbar ist, da Tetrafluorethen unter Druck zu brisanten Explosionen neigt (H. Liebig u. K. Ulm, Chemiker-Zeitung 99 (1975) 477). Ein weiteres Verfahren beinhaltet die Umwandlung von α-Hydroxycarbonsäuren mit dem toxischen und schwer handhabbaren Schwefeltetrafluorid in α-Trifluormethyl-Alkohole. Auf diese Weise ist z.B. das 1,1,1,4,4,4-Hexafluor-2-butanol hergestellt worden (A. I. Burmakov et al, Zh. Org. Khim. 16, 1401 (1980)). In der DE-AS 1 244 169 wird die Umsetzung bestimmter dichlorperhalogenierter Cycloalkene, worin 2 Chloratome an den Kohlenstoffatomen der Doppelbindung stehen, mit Alkalialkoxyd in Alkanolen zu den entsprechenden methoxy-perhalogenierten Cycloalkenen beschrieben.

Es besteht deshalb das Bedürfnis nach einem einfach durchführbaren allgemeinen Verfahren zur Herstellung teilfluorierter Alkohole, das auch in größerem Maßstab anwendbar ist.

Es wurde nun ein vorteilhaftes allgemeines Verfahren zur Herstellung teilfluorierter Alkohole der Formel (I), 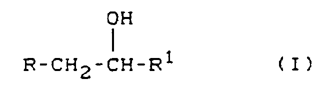
in welcher
- R und R¹: gleich oder verschieden sind und für einfach oder mehrfach durch Fluor substituiertes Alkyl, für einfach oder mehrfach durch Fluor substituiertes Cycloalkyl steht oder R und R¹ zusammen die Gruppe -(CF₂)ₙ- darstellen, wobei n für 2, 3 oder 4 steht,

gefunden, das dadurch gekennzeichnet ist, daß man
a) Verbindungen der Formel (II), 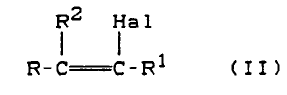 in welcher
   - R und R¹: die oben angegebene Bedeutung haben,
   - R²: für Wasserstoff, Fluor, Chlor oder Brom steht und
   - Hal: für Halogen steht,
   mit einem Alkohol der Formel (III),

   HO-R³ (III)

   in welcher
   - R³: für gegebenenfalls substituierte Reste aus der Reihe Alkyl, Alkenyl, Cycloalkyl, Cycloalkenyl, Aryl, Heteroaryl und Aralkyl steht,
   in Gegenwart einer Base zu Verbindungen der Formel (IV), 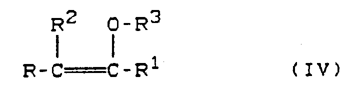 in welcher
   - R, R¹, R² und R³: die oben angegebene Bedeutung haben,
   umsetzt, und
b) die Verbindungen der Formel (IV) , gegebenenfalls nach ihrer Isolierung, katalytisch mit Wasserstoff,

entweder
α) in Abwesenheit einer Base zu Verbindungen der Formel (I) hydriert,
oder
β) in Gegenwart einer Base zu Verbindungen der Formel (V), 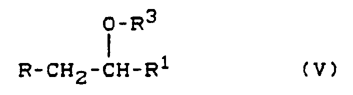
in welcher
- R, R¹ und R³: die oben angegebene Bedeutung haben,

hydriert und die Verbindungen der Formel (V), gegebenenfalls nach ihrer Isolierung, mit etherspaltenden Mitteln in Verbindungen der Formel (I) überführt.

Es ist ausgesprochen überraschend, daß das erfindungsgemäße Verfahren so einfach und gut durchführbar ist. Besonders stellt es einen allgemeinen Zugang zu Verbindungen der Formel (I) dar, die bisher in technischen Mengen nicht verfügbar waren. Im Einzelnen ist überraschend, daß man Verbindungen der Formel (IV) in so guter Ausbeute und Reinheit erhalten kann, selbst wenn R² für Halogen steht. Denn es war zu erwarten, daß zusätzlich auch das Halogen-Atom (R²) gegen OR³ substituiert wird und gegebenenfalls eines oder zwei allylisch ständige Fluoratome substituiert werden (J.D. Park et al , J. Am. Chem. Soc. 73 (1951) 2342). Weiterhin ist es überraschend, daß bei den Hydrierungen nicht die allylisch ständigen Fluor-Atome hydrogenolytisch entfernt werden, wie es z.B. nach der Literatur zu erwarten gewesen wäre (vergl. M. Hudlicky, J. Fluorine Chem. 44, 345 (1989)). Es war weiterhin nicht zu erwarten, daß die Etherspaltung so selektiv zu den teilfluorierten Alkoholen der Formel (I) führt und keine konkurrierende Eliminierung stattfindet. Dabei ist es besonders überraschend, daß die Hydrierung und die Etherspaltungen bei Stufe b-Variante α als "Eintopf-Reaktionen" durchgeführt werden können, obwohl zu erwarten war, daß die bei der Hydrierung freiwerdende Halogenwasserstoffsäure auch Verbindungen der Formel (IV) spalten würde.

In den Formeln (I) bis (V) stehen R und R¹ unabhängig voneinander vorzugsweise für einfach oder mehrfach durch Fluor substituiertes Alkyl mit 1 bis 10, vorzugsweise 1 bis 8, insbesondere 1 bis 4 Kohlenstoffatomen, für einfach oder mehrfach durch Fluor substituiertes Cycloalkyl mit 3 bis 10, vorzugsweise 3 bis 6, insbesondere 3 bis 5 Kohlenstoffatomen oder R und R¹ stehen zusammen für die Gruppe -(CF₂)-ₙ, wobei n für 2, 3 oder 4 steht, vorzugsweise n für 2 oder 3 und insbesondere n für 2 steht.

Für Alkyl in R und R¹ seien beispielhaft Methyl, Ethyl, n- oder iso-Propyl, n-, iso-, sec.- oder tert.-Butyl genannt. Die Substitution von Alkyl durch Fluor ist vorzugweise einfach bis neunfach, insbesondere einfach bis fünffach. Insbesondere sei Trifluormethyl genannt.

Für Cycloalkyl in R und R¹ seien beispielhaft Cyclopropyl, Cyclopentyl oder Cyclohexyl genannt. Der Rest Cycloalkyl in R und R¹ ist im Falle der Substitution vorzugsweise einfach bis zwölffach, insbesondere einfach bis sechsfach, ganz besonders einfach bis vierfach durch Fluor substituiert.

Der Rest Hal in der Formel (II) steht vorzugsweise für Fluor, Chlor oder Brom, insbesondere für Fluor oder Chlor.

Der Rest R² in den Formeln II und IV steht für Wasserstoff, Fluor, Chlor oder Brom, insbesondere für Wasserstoff, Fluor oder Chlor.

Der Rest R³ in den Formeln (III) und (V) steht vorzugsweise für jeweils gegebenenfalls substituiertes Alkyl mit 1 bis 8, insbesondere 1 bis 5 Kohlenstoffatomen, Alkenyl mit 3 bis 10, insbesondere 3 bis 7 Kohlenstoffatomen, Cycloalkyl mit 3 bis 6 Kohlenstoffatomen wie Cyclopropyl, Cyclopentyl oder Cyclohexyl, Cycloalkenyl mit 4 bis 10, insbesondere 4 bis 7 Kohlenstoffatomen, Phenyl, Naphthyl, Pyridyl, Pyrimidyl oder Phenylalkyl mit 1 bis 4, insbesondere 1 oder 2 Kohlenstoffatomen im Alkylteil. Als Substituenten sind ausgewählt Halogen, insbesondere Fluor und Chlor, Alkoxy mit 1 bis 10, vorzugsweise 1 bis 6, insbesondere 1 bis 4 Kohlenstoffatomen, beispielsweise Methoxy, Ethoxy, n- oder i-Propoxy sowie 1 oder 2 Oxo-Gruppen.

Insbesondere steht R³ in den Formeln (III) und (V) für primäres, sekundäres oder tertiäres, vorzugsweise für sekundäres oder tertiäres, Alkyl mit 3 bis 6 Kohlenstoffatomen, für Cyclohexyl, Benzyl oder Phenyl. Für Alkyl in R³ sei beispielhaft Methyl, Ethyl, n-Propyl, i-Propyl, n-Butyl, s-Butyl und t-Butyl genannt. Ganz besonders bevorzugt steht R³ für i-Propyl, Phenyl, Benzyl oder Cyclohexyl.

Besonders vorteilhaft lassen sich nach dem erfindungsgemäßen Verfahren teilfluorierte Alkohole der Formel (I) herstellen, bei welchen R und R¹ zusammen die Gruppe -(CF₂)ₙ- darstellen, wobei n für 2, 3 oder 4, vorzugsweise n für 2 oder 3 und insbesondere n für 2 steht.

Der Verlauf des erfindungsgemäßen Verfahrens kann durch das folgende Formelschema veranschaulicht werden:

### Stufe a:

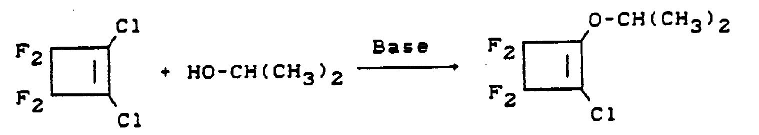

### Stufe b (Variante α):

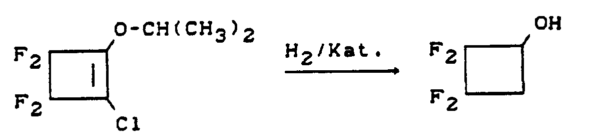

### Stufe b (Variante β):

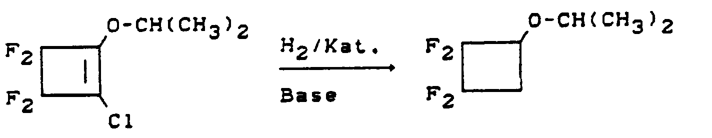

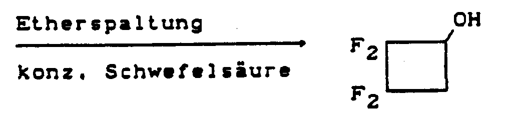

Die zur Durchführung des erfindungsgemäßen Verfahrens benötigten Verbindungen der Formeln (II) und (III) sind bekannte Synthesechemikalien oder lassen sich nach bekannten Methoden herstellen.

Die Umsetzung des Alkens der Formel (II) mit einem Alkohol der Formel (III) (Stufe a) wird in Gegenwart einer Base und gegebenenfalls in Gegenwart eines Verdünnungsmittels durchgeführt.

Als Base kommen dafür Alkali- und Erdalkalihydroxide, tertiäre Amine oder Alkoholate in Frage. Bevorzugt sind Alkali- und Erdalkalihydroxide und tert. Amine. Besonders bevorzugt sind NaOH und Triethylamin.

Als Verdünnungsmittel kommen bei Stufe a alle inerten organischen Lösungsmittel in Betracht. Beispiele hierfür sind Alkylether, Kohlenwasserstoffe, teil- oder perhalogenierte Kohlenwasserstoffe sowie gegebenenfalls substituierte aromatische Kohlenwasserstoffe. Der eingesetzte Alkohol der Formel (III) kann auch selbst als Lösungsmittel dienen. Gegebenenfalls kann auf Verdünnungsmittel ganz verzichtet werden.

Die Reaktionstemperaturen können bei der Durchführung des erfindungsgemäßen Verfahrens (Stufe a) in einem größeren Bereich variiert werden. Im allgemeinen arbeitet man bei Temperaturen zwischen -50°C und +100°C, vorzugweise bei Temperaturen zwischen -20°C und +50°C, insbesondere zwischen -20°C und +30°C bzw. Raumtemperatur.

Das erfindungsgemäße Verfahren wird im allgemeinen bei Normaldruck durchgeführt, es kann aber auch bei erhöhtem oder vermindertem Druck durchgeführt werden.

Das Verhältnis der Einsatzmaterialien der Formeln (II) und (III) bei der Durchführung des erfindungsgemäßen Verfahrens (Stufe a) ist nicht kritisch und kann 1:0,5 bis 1:10 betragen. Bevorzugt sind Verhältnisse von 1:0,9 bis 1:1,5.

Die Umsetzungen der Verbindungen der Formel (IV) gemäß Stufe (b) werden, gegebenenfalls nach ihrer Isolierung, mit Wasserstoff in Gegenwart eines Katalysators und gegebenenfalls in Gegenwart eines Verdünnungsmittels entweder in Abwesenheit einer Base direkt zu den Verbindungen der Formel (I) hydriert (Stufe b-Variante α) oder in Gegenwart einer Base zunächst zu Verbindungen der Formel (V) umgesetzt (Hydrierung der Doppelbindung) und dann mit etherspaltenden Mitteln in Verbindungen der Formel (I) überführt (Stufe b-Variante β).

Geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxidhydrate handeln. Zusätzlich können die Metalle, Kupfer, Vanadin, Molybdän, Chrom und/oder Mangan, sowie Verbindungen dieser Metalle zugegen sein.

Die Hydrierkatalysatoren können ausschließlich oder überwiegend aus wasserstoffübertragenden Substanzen bestehen, diese können aber auch auf Trägermaterialien aufgebracht sein. Als Trägermaterialien für die Wasserstoff-übertragenden Substanzen kommen beispielsweise in Frage: anorganische Materialien wie Kieselgur, Kieselsäure, Aluminiumoxide, Alkali- und Erdalkalisilikate, Aluminiumsilikate, Montmorillonit Zeolithe, Spinelle, Dolomit, Kaolin, Magnesiumsilikate, Zirkonoxid, Zinkoxid, Calciumcarbonat, Siliciumcarbid, Aluminiumphosphate, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien. Das Trägermaterial kann beispielsweise in Form von Kugeln, Strängen, Fäden, Zylindern, Polygonen oder in Pulverform vorliegen.

Derartige Trägerkatalysatoren können im allgemeinen 0,5 bis 50 Gew.-%, vorzugsweise 1 bis 10 Gew.-% der wasserstoffübertragenden Substanz, bezogen auf die Gesamtmasse des Trägerkatalysators, enthalten. Die wasserstoffübertragende Substanz kann dabei homogen im Trägermaterial verteilt sein, bevorzugt sind jedoch Katalysatoren, in deren äußerer Schicht oder auf deren Oberfläche die wasserstoffübertragende Substanz abgelagert ist. Die Herstellung und die Formgebung von Katalysatoren, die im erfindungsgemäßen Verfahren Verwendung finden können, kann in bekannter Weise erfolgen (siehe beispielsweise Houben-Weyl, Methoden der organischen Chemie, Band IV, 1c, Teil I, S. 16-26, Georg Thieme Verlag, Stuttgart 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid.

Bevorzugte Hydrierkatalysatoren, die ausschließlich oder überwiegend aus wasserstoffübertragender Substanz bestehen, sind beispielsweise oxidische Katalysatoren wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiveren Metallen herstellbare Schwarzkatalysatoren, wie Palladium/Schwarz, Platin/Schwarz und Rhodium/Schwarz.

Besonders bevorzugte Katalysatoren für das erfindungsgemäße Verfahren (Stufe b) sind Palladium auf Kohle, Palladium auf Aluminiumoxid, Palladium auf Kieselsäure und Palladium auf Calciumcarbonat.

Der Hydrierkatalysator kann in das erfindungsgemäße Verfahren beispielsweise in Mengen von 0,05 bis 50 Gew.-% wasserstoffübertragende Substanz, bezogen auf das Gesamtgewicht des Reaktionsgemisches, eingesetzt werden. Vorzugsweise beträgt diese Menge 0,1 bis 10 Gew.-%.

Zur Durchführung des erfindungsgemäßen Verfahrens können auch Gemische aus zwei oder mehreren der genannten Hydrierkatalysatoren verwendet werden und gegebenenfalls wird während der Reaktion Katalysator nachgesetzt.

Das erfindungsgemäße Verfahren (Stufe b) kann in Abwesenheit eines Verdünnungsmittels in flüssiger Phase durchgeführt werden. Vorzugsweise wird die Umsetzung jedoch unter Verwendung eines Verdünnungsmittels durchgeführt. Als Verdünnungsmittel kommen alle unter den Reaktionsbedingungen inerten organischen Lösungsmittel in Frage, beispielsweise aliphatische und cycloaliphatische Kohlenwaserstoffe, wie Hexan, Heptan, Octan, Cyclohexan, Methylcyclohexan, Decalin; aliphatische oder alicyclische Ether, wie Diethylether, Dipropylether, Diisopropylether, Dibutylether, Methylbutylether, Ethylbutylether, Ethylenglykoldimethylether, 1,3-Dioxolan, 1,4-Dioxan, Tetrahydrofuran; niedrige aliphatische Alkohole, wie Methanol, Ethanol, Propanol, Isopropanol, Butanol, Isobutanol, tert.-Butanol, Ethylenglykol; Etheralkohole, wie Diethylenglykol, Ethylenglykolmonomethylether, Ethylenglykolmonobutylether, Dipropylenglykol. Ebenso ist Wasser einsetzbar. Daneben können Alkancarbonsäuren, wie Essigsäure, Propionsäure, Hexansäure etc. verwendet werden.

Auch Gemische aus 2 oder mehreren der vorgenannten Lösungsmittel können verwendet werden.

Bevorzugte Lösungsmittel sind cycloaliphatische Kohlenwasserstoffe oder aliphatische Ether, insbesondere Cyclohexan, Methyl-cyclohexan, Tetrahydrofuran, 1,4-Dioxan, Wasser und Essigsäure.

Falls bei Stufe b eine Base verwendet wird, eignen sich z.B. Alkali- und Erdalkalihydroxide und -carbonate, tertiäre organische Amine oder Alkoholate. Bevorzugt sind Alkali- und Erdalkalihydroxide und -carbonate sowie tertiäre Amine. Besonders bevorzugt sind Natrium- und Kaliumhydroxid, Natrium- und Kaliumcarbonat, Triethylamin und Pyridin.

Das erfindungsgemäße Verfahren (Stufe b) kann in einem weiten Temperaturbereich durchgeführt werden. Im allgemeinen erfolgt die Umsetzung gemäß Stufe b bei Temperaturen von 0 bis 300°C. Bevorzugt sind Temperaturen von 20 bis 230°C, insbesondere Temperaturen von 60°C bis 220°C.

Der Wasserstoffdruck bei der Hydrierung ist nicht kritisch und kann z.B. von 1 bis 300 bar reichen. Es ist gegebenenfalls vorteilhaft, verbrauchten Wasserstoff durch weitere Zusetzung auszugleichen. Bevorzugt sind Wasserstoffdrücke von 5 bis 250 bar, insbesondere von 20 bis 200 bar.

In einer besonderen Ausführungsform des erfindungsgemäßen Verfahrens (Stufe b-Variante α) werden Verbindungen der Formel (IV), gegebenenfalls in Gegenwart eines Verdünnungsmittels, ohne Zusatz einer Base zu Verbindungen der Formel (I) umgesetzt.

Eine weitere besondere Ausführungsform des erfindungsgemäßen Verfahrens beinhaltet die katalytische Hydrierung von Verbindungen der Formel (IV), gegebenenfalls in Gegenwart eines Verdünnungsmittels, mit Zusatz einer Base zu Verbindungen der Formel (V) (Stufe b-Variante ß). Diese werden, gegebenenfalls nach ihrer Isolierung, mit etherspaltenden Mitteln in Verbindungen der Formel (I) überführt.

Bei der Hydrierung kann bei aromatischen Resten von R³, der aromatische Rest mithydriert werden. Auf diese Weise kann die Bedeutung von R³ als beispielsweise Phenyl zu Cyclohexyl geändert werden (vgl. Herstellungsbeispiel 9).

Als etherspaltende Mittel können bei Stufe b, Variante ß die allgemein bekannten Reagentien verwendet werden, z.B. Brönsted- und Lewis-Säuren. Beispiele hierfür sind Mineralsäuren wie Salzsäure, Fluorwasserstoffsäure, Bromwasserstoffsäure, Jodwasserstoffsäure oder Schwefelsäure, HBF₄, CF₃COOH, bzw. BF₃, FeCl₃, AlCl₃, BCl₃, BBr₃, BJ₃. Auch sogenannte "weiche" Nucleophile, wie z.B. Jodid (beispielsweise als Lithium oder Kalium-Salz) oder Alkylsulfide können als etherspaltende Mittel verwendet werden. Bevorzugt sind starke Brönsted-Säuren und Lewis-Säuren. Besonders bevorzugt sind HCl, Schwefelsäure, FeCl₃ und BF₃.

Für die Etherspaltung geeignete Temperaturen sind beispielsweise 0 bis 300°C. Die Etherspaltung kann gegebenenfalls in Gegenwart eines Verdünnungsmittels erfolgen. Bevorzugt ist, die Spaltung in Abwesenheit von Verdünnungsmitteln durchzuführen.

Die nach dem erfindungsgemäßen Verfahren herstellbaren teilfluorierten Alkohole der Formel (I) sind Ausgangsstoffe zur Synthese von biologisch aktiven Substanzen wie z.B. zur Herstellung von Phosphorsäureester, welche zur Schädlingsbekämpfung eingesetzt werden können (vgl. DE-A-38 19 632 vom 09. Juni 1988).

So läßt sich beispielsweise Thiophosphorsäure-S-propyl-O-ethyl-O-(3,3,2,2-tetrafluorcyclobutyl)triester der Formel 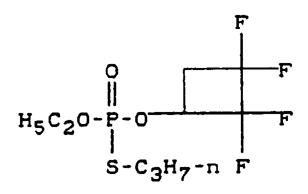
welcher u.a. insektizide oder nematizide Wirkung hat, folgendermaßen herstellen:

289,5 g (1,5 Mol) Thiophosphorsäure-dichlorid-S-Propylester werden in 4,5 l Toluol gelöst und bei 0 bis 5°C eine Mischung von 69,1 g Ethanol (1,5 Mol), 210 ml Triethylamin und 250 ml Toluol zu dieser Lösung getropft (ca. 25 min). Nach 5,5 Stunden bei 0°C werden 25 g DABCO (Diazabicyclounden) und 210 ml Triethylamin zur Reaktionsmischung gegeben und zwischen 5 und 10°C eine Mischung von 216 g (1,5 Mol) 2,2,3,3-Tetrafluorcyclobutanol-1 und 800 ml Toluol zugetropft. Anschließend wird 18 Stunden bei 20°C, dann 3 Stunden bei 60°C nachgerührt, auf 20°C abgekühlt, mit 3 l Wasser versetzt und die organische Phase abgetrennt. Die Toluolphase wird dann zunächst mit 2 l 1N Salzsäure, dann mit 2 l 1N Natronlauge und abschließend mit 3 l Wasser gewaschen, mit Magnesiumsulfat getrocknet und das Lösungsmittel im Vakuum abdestilliert. Nach Destillation des Rückstandes in einem Dünnschichtverdampfer (0,1 mbar) erhält man 330 g Thiophosphorsäure-O-(2,2,3,3-tetrafluor-cyclobutylester)-O-ethylester-S-propylester in einer Reinheit von 91 %. (65 % der Theorie).

Das erfindungsgemäße Verfahren wird durch folgende Beispiele veranschaulicht:

### Beispiel 1 / Verbindung IV-1

Darstellung von 2-Chlor-3,3,4,4-tetrafluorcyclobutenylisopropylether / Stufe a

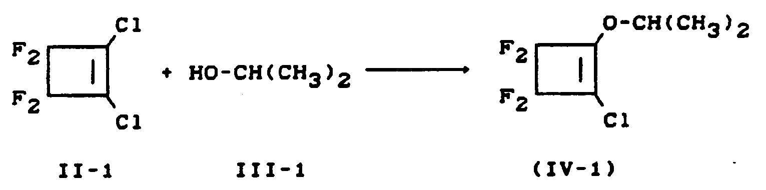

Zu einer Lösung aus 786 g (3,94 Mol) 1,2-Dichlor-3,3,4,4-tetrafluorcyclobuten, 328 g (5,47 Mol) technischem Isopropanol werden bei -10°C 200 g (5 Mol) gepulvertes Natriumhydroxid dosiert. Es wird noch zwei Stunden nachgerührt, der Feststoff abgesaugt und das Filtrat destilliert.
- Kp.: : 47 - 48°C (24 Torr)
- Ausbeute: : 747 g (85% d. Theorie)
- Reinheit: : 98%
- IR (Film): : 1690 cm⁻¹ (C=C)
- ¹H-NMR: : 1,42 ppm (d, 6H), 4,87 ppm (hept., 1H)
- ¹⁹ F-NMR: : -37 ppm (m, 2F), -39 ppm (m, 2F)

### Beispiel 2 / Verbindung IV-2

Darstellung von 2,3,3,4,4-Pentafluorcyclobutenyl-isopropylether/Stufe a

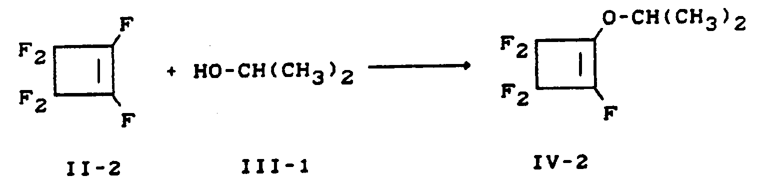

Zu einer Lösung aus 200 g (1,23 Mol) 1,2,3,3,4,4-Hexafluorcyclobuten und 90 g (1,5 Mol) Isopropanol in 800 ml Diethylether werden bei Raumtemperatur 70 g (1,23 Mol) gepulvertes Kaliumhydroxid dosiert. Man rührt noch eine Stunde und arbeitet wie in Beispiel 1 beschrieben auf. Das Produkt wird anschließend destilliert.
- Kp.: : 64 - 66°C (140 Torr)
- Ausbeute: : 152 g (67% der Theorie)
- Reinheit: : 98%
- IR: : 1760 cm⁻¹ (C=C)
- MS: : 202 (M)

### Beispiel 3 / Verbindung IV-3

Darstellung von 2-Chlor-3,3,4,4,5,5-hexafluorcyclopentenylphenylether / Stufe a

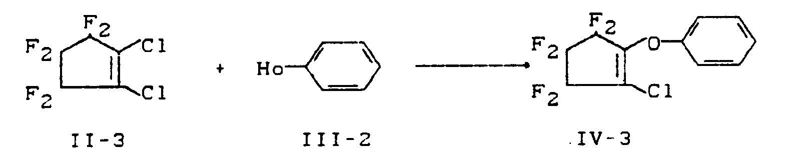

Analog Beispiel 1 wird aus 1,2-Dichlor-3,3,4,4,5,5-hexafluorcyclopenten und Phenol 2-Chlor-3,3,4,4,5,5-hexafluorcyclopentenyl-phenylether erhalten. Ausbeute (67% der Theorie; Kp. 151-153°C).

### Beispiel 4 / Verbindung IV-4

Darstellung von 2-(1,1,1,4,4,4-Hexafluorbut-2-enyl)-benzylether / Stufe a

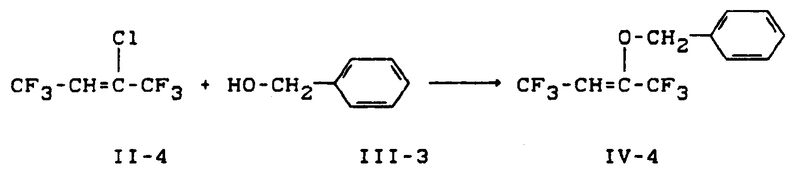

Zu einer Mischung aus 350 g (8,75 Mol) festem Natriumhydroxid und 500 g (4,63 Mol) Benzylalkohol tropft man bei 20 - 40°C 893 g 2-Chlor-1,1,1,4,4,4-hexafluorbuten-2. Man rührt noch zwei Stunden bei 50°C, gießt auf Wasser und extrahiert mit Dichlormethan. Die organischen Phasen werden getrocknet und durch Destillation erhält man 2-(1,1,1,4,4,4-Hexafluorbut-2-enyl)-benzylether.
- Ausbeute: : 1076 g (89% der Theorie)
- Kp. (18 mbar): : 70 - 72°C.

### Beispiel 5 / Verbindung V-1

Darstellung von 9,2,3,3-Tetrafluorcyclobutylisopropylether / Stufe b (Variante β)

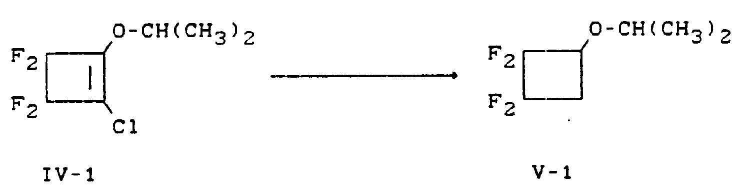

Eine Lösung aus 274 g (1,26 Mol) 2-Chlor-3,3,4,4-tetrafluorcyclobutenyl-isopropylether, 252 g (2,50 Mol) Triethylamin und 1200 ml über Calciumchlorid getrockneten Ether werden mit 40 g 10%iger Palladiumkohle unter Stickstoff versetzt in einem 3 l VA-Autoklaven bei 100°C bis zur Druckkonstanz hydriert (ca. 5 Stunden). Es wird vom Feststoff abgesaugt, gut mit Ether nachgewaschen und das Filtrat mit verdünnter Salzsäure gewaschen. Nach dem Trocknen wird destilliert.
- Kp.: : 121°C
- Ausbeute: : 198 g (86% der Theorie)
- Reinheit: : 98%
- ¹⁹F-NMR: : -30 ppm (m, 2F), -39 ppm (m, 2F)
- MS: : 186 (M⁺), 171 (M⁺-Me)

### Beispiel 6 / Verbindung V-1

Darstellung von 2,2,3,3-Tetrafluorcyclobutyl-isopropylether / Stufe b (Variante β)

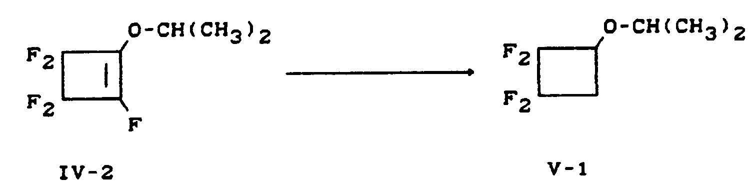

280 g (1,39 Mol) 2,3,3,4,4-Pentafluorcyclobutenyl-isopropylether werden in 1200 ml Ether wie in Beispiel 5 beschrieben hydriert und aufgearbeitet. Man erhält so 2,2,3,3-Tetrafluorcyclobutyl-isoproyplether in einer Ausbeute von 66 % der Theorie.

### Beispiel 7 / Verbindung V-1

Darstellung von 2,2,3,3-Tetrafluorcyclobutyl-isopropylether / Stufe b (Variante β)

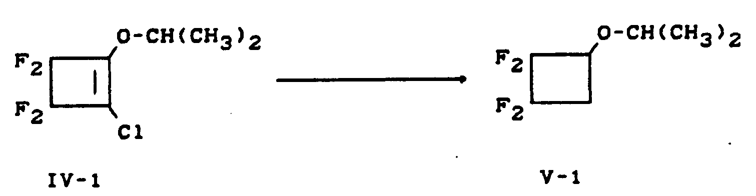

30 g (0,132 Mol) 2-Chlor-3,3,4,4-tetrafluorcyclobutenyl-isopropylether und 20 g (0,145 Mol) Kaliumcarbonat werden in 100 ml Wasser über 10 g Palladium-Kohle bei 120°C fünf Stunden hydriert. Es wird vom Katalysator abfiltriert und das Filtrat mit Dichlormethan extrahiert. Nach dem Trocknen erhält man durch Destillation 2,2,3,3-Tetrafluorcyclobutyl-isopropylether mit einer Ausbeute von 55 %.

### Beispiel 8 / Verbindung V-1

Darstellung von 2,2,3,3-Tetrafluorcyclobutyl-isopropylether / Stufe b (Variante β)

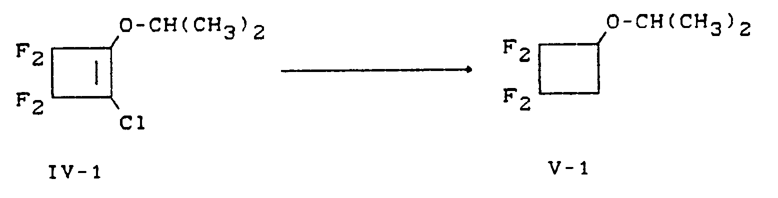

Verwendet man statt Kaliumcarbonat und Wasser käufliche Pufferlösung pH 9 (Borat-Salzsäure) und verfährt sonst wie in Beispiel 7 beschrieben, erhält man 2,2,3,3-Tetrafluorcyclobutyl-isopropylether mit 58 % Ausbeute.

### Beispiel 9 / Verbindung V-2

Darstellung von 2,2,3,3,4,4-Hexafluorcyclopentyl-cyclohexylether / Stufe b (Variante β)

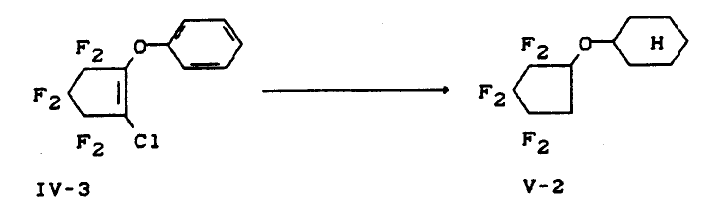

In einem 1,3 l Autoklaven werden 605 g (2 Mol) 2-Chlor-3,3,4,4,5,5-hexafluorcyclopentenyl-phenylether in 500 ml pH 9-Puffer (Borat/HCl) supsendiert und über 50 g Palladium-Kohle bei 70 - 80°C mit 80 bar Wasserstoff hydriert. Es wird analog Beispiel 7 aufgearbeitet und man erhält 2,2,3,3,4,4-Hexafluorcyclopentyl-cyclohexylether.
- Kp.: : 86 - 89°C
- Ausbeute: : 502 g (91 % der Theorie)
- ¹H-NMR: : 1,08-1,54 ppm (m, 6H); 1,83 ppm (m, 4H); 2,34 ppm (m, 1H); 2,71 ppm (m, 1H); 3,48 ppm (m, 1H); 4,20 ppm (m, 1H)
- ¹⁹F-NMR (gegen CF₃COOH): : -33,5 ppm (m, 2F); -47,3 ppm (m, 2F); -55,0 ppm (m, 2F)

### Beispiel 10 / Verbindung V-3

Darstellung von 2,2,3,3,4,4-Hexafluorcyclopentyl-isopropylether / Stufe b (Variante β)

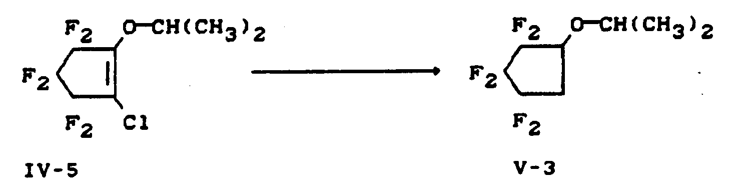

Analog Beispiel 8 werden 537 g (2 Mol) 2-Chlor-3,3,4,4,5,5-hexafluorcyclopentenyl-isopropylether hydriert und aufgearbeitet. Man erhält 2,2,3,3,4,4-Hexafluorcyclopentyl-isopropylether.
- Ausbeute: : 419 g (89 % der Theorie)
- Kp.: : 142 - 144°C
- ¹H-NMR: : 1,2 ppm (m, 6H); 2,34 ppm (m, 1H); 2,71 pppm (m, 1H); 3,82 ppm (m, 1H); 4,13 ppm (m, 1H)

### Beispiel 11 / Verbindung I-1

Darstellung von 1,1,1,4,4,4-Hexafluorbutan-2-ol / Stufe b (Variante α) / Eintopfreaktion

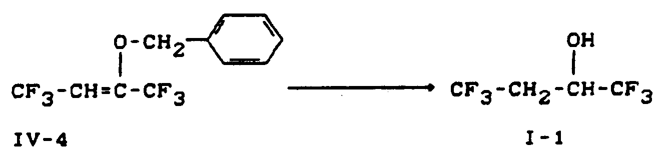

In einem 300 ml Autoklaven werden 27,0 g (0,1 Mol) 2-(1,1,1,4,4,4-Hexafluorbut-2-enyl)-benzylether in 150 ml Diglyme über 5 g 5 % Palladium-Kohle mit 30 - 40 bar Wasserstoff bei Raumtemperatur hydriert. Das 1,1,1,4,4,4-Hexafluorbutan-2-ol erhält man durch Destillation.
- Ausbeute: : 11,2 g (62% der Theorie)
- Kp.: : 87°C
- MS (CI): : 183 (M⁺+H)
- IR: : 3400 - 3100 cm⁻¹ (OH); 1160, 1120, 1020 cm⁻¹ (CF₃)

### Beispiel 12 / Verbindung I-2

Darstellung von 2,2,3,3-Tetrafluorcyclobutanol / Stufe b (Variante α) / Eintopfreaktion

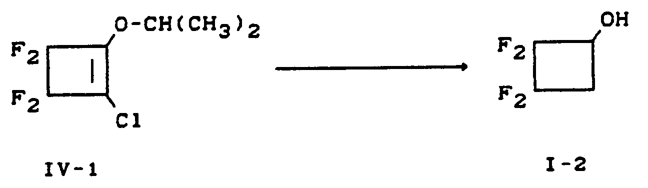

50 g (0,23 Mol) 2-Chlor-3,3,4,4-tetrafluorcyclobut-2-enyl-isopropylether in 150 ml Cyclohexan werden mit 2,5 g 10 %iger Palladium-Kohle versetzt und bei einem Wasserstoffdruck von 100 bar 10 Stunden auf 200°C erhitzt. Es wird filtriert und destilliert. Man erhält reines 2,2,3,3-Tetrafluorcyclobutanol.
- Kp.: : 121 - 122°C
- Reinheit: : 99 %
- Ausbeute: : 18,6 g (56 % der Theorie)

### Beispiel 13 / Verbindung I-2

Darstellung von 2,2,3,3-Tetrafluorcyclobutanol / Stufe b (Variante β) - Etherspaltung

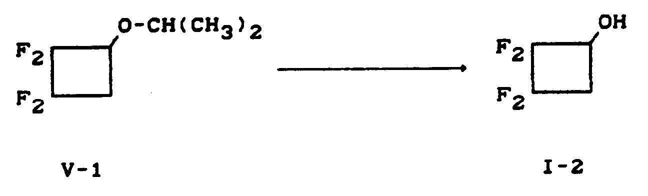

473 g (2,54 Mol) 2,2,3,3-Tetrafluorcyclobutyl-isopropylether werden mit 8 g konz. Schwefelsäure bis zum Ende der Gasentwicklung (Propen) bei 140°C gerührt (ca. 14 Stunden). Das Produkt wird anschließend direkt abdestilliert.
- Kp.: : 120 - 121 °C
- Ausbeute: : 345 g (96 % der Theorie)
- Reinheit: : 98 % / Wassergehalt 1 - 2 % (Karl-Fischer)

### Beispiel 14 / Verbindung I-3

Darstellung von 2,2,3,3,4,4-Hexafluorcyclopentanol / Stufe b (Variante β) - Etherspaltung

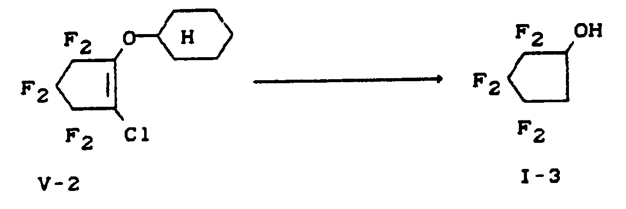

276 g (1 Mol) 2,2,3,3,4,4-Hexafluorcyclopentyl-cyclohexylether werden analog Beispiel 13 umgesetzt und aufgearbeitet. Man erhält durch Destillation 2,2,3,3,4,4-Hexafluorcyclopentanol.
- Ausbeute: : 165 g ( 85 % der Theorie)
- Kp.: : 130 - 132°C
- ¹H-NMR: : 2,39 ppm, (m, 1H); 2,72 ppm (m, 1H); 3,27 ppm (s, 1H), 4,38 ppm (m, 1H)
- ¹⁹F-NMR (gegen CF₃COOH): : -32,2 ppm (m, 2F); -49,7 ppm (m, 2F); -54,2 ppm (m, 2F)

## Patentansprüche

1. Verfahren zur Herstellung teilfluorierter Alkohole der Formel (I), 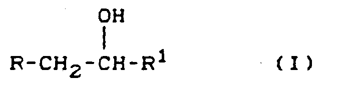 in welcher
R und R¹ gleich oder verschieden sind und für einfach oder mehrfach durch Fluor substituiertes Alkyl, für einfach oder mehrfach durch Fluor substituiertes Cycloalkyl steht oder R und R¹ zusammen die Gruppe -(CF₂)ₙ- darstellen, wobei n für 2, 3 oder 4 steht,
dadurch gekennzeichnet, daß man
a) Verbindungen der Formel (II), 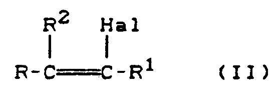 in welcher
R und R¹ die oben angegebene Bedeutung haben,
R² für Wasserstoff, Fluor, Chlor oder Brom steht und
Hal für Halogen steht,
mit einem Alkohol der Formel (III),
HO-R³ (III)
in welcher
R³ für gegebenenfalls substituierte Reste aus der Reihe Alkyl, Alkenyl, Cycloalkyl, Cycloalkenyl, Aryl, Heteroaryl und Aralkyl steht,
in Gegenwart einer Base zu Verbindungen der Formel (IV), 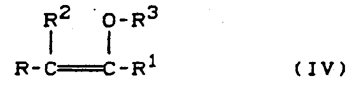 in welcher
R, R¹, R² und R³ die oben angegebene Bedeutung haben,
umsetzt, und
b) die Verbindungen der Formel (IV), gegebenenfalls nach ihrer Isolierung, katalytisch mit Wasserstoff,
entweder
α) in Abwesenheit einer Base zu Verbindungen der Formel (I) hydriert,
oder
β) in Gegenwart einer Base zu Verbindungen der Formel
(V), 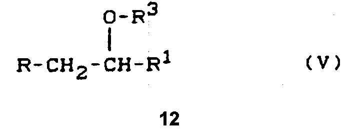 in welcher
R, R¹ und R³ die oben angegebene Bedeutung haben,
hydriert und die Verbindungen der Formel (V), gegebenenfalls nach ihrer Isolierung, mit etherspaltenden Mitteln in Verbindungen der Formel (I) überführt.

2. Verfahren gemäß Anspruch 1, dadurch gekennzeichnet, daß bei Stufe a Alkali- oder Erdalkalihydroxide, tertiäre Amine oder Alkoholate als Base eingesetzt werden.

3. Verfahren gemäß Anspruch 1, dadurch gekennzeichnet, daß Stufe a in Gegenwart von Verdünnungsmitteln oder einem Überschuß des Alkohols der Formel (III) durchgeführt wird.

4. Verfahren gemäß Anspruch 1, dadurch gekennzeichnet, daß Stufe b in Gegenwart von Verdünnungsmitteln durchgeführt wird.

5. Verfahren gemäß Anspruch 1, dadurch gekennzeichnet, daß bei Stufe b-β Alkali- und Erdalkalihydroxide, Erdalkalicarbonate, tertiäre organische Amine oder Alkoholate verwendet werden.

6. Verfahren gemäß Anspruch 1, dadurch gekennzeichnet, daß die Stufe b-α und b-β (Hydrierung und Etherspaltung) als Eintopfreaktion durchgeführt werden.

7. Verfahren gemäß Anspruch 1, dadurch gekennzeichnet, daß R und R¹ für einfach oder mehrfach durch Fluor substituiertes Alkyl mit 1 bis 10 Kohlenstoffatomen, für einfach oder mehrfach durch Fluor substituiertes Cycloalkyl mit 3 bis 10 Kohlenstoffatomen stehen oder R und R¹ zusammen für die Gruppe -(CF₂)ₙ- stehen, wobei n für 2, 3 oder 4 steht.

8. Verfahren gemäß Anspruch 1, dadurch gekennzeichnet, daß R³ für jeweils gegebenenfalls substituiertes Alkyl mit 1 bis 8 Kohlenstoffatomen, Alkenyl mit 3 bis 8 Kohlenstoffatomen, Cycloalkyl mit 3 bis 6 Kohlenstoffatomen, Cycloalkenyl mit 4 bis 10 Kohlenstoffatomen, Phenyl, Naphthyl, Pyridyl, Pyrimidyl oder Phenylalkyl mit 1 bis 4 Kohlenstoffatomen im Alkylteil steht.

9. Verfahren gemäß Anspruch 8, dadurch gekennzeichnet, daß als Substituenten Halogen, Alkoxy mit 1 bis 4 Kohlenstoffatomen sowie 1 oder 2 Oxo-Gruppen ausgewählt sind.

10. Verfahren zur Herstellung der Verbindungen der Formel (I), 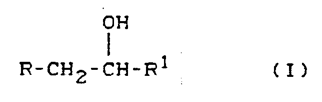 in welcher
R und R¹ die in Anspruch 1 angegebene Bedeutung haben,
dadurch gekennzeichnet, daß man
die Verbindungen der Formel (IV), 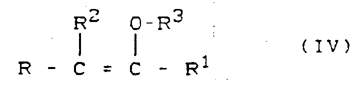 in welcher
R, R¹ , R² und R³ die in Anspruch 1 angegebene Bedeutung haben, katalytisch mit Wasserstoff,
entweder
α) in Abwesenheit einer Base zu Verbindungen der Formel (I) hydriert,
oder
β) in Gegenwart einer Base zu Verbindungen der Formel (V), 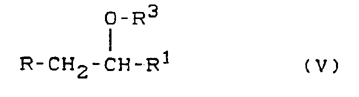 in welcher
R R¹ und R³ die in Anspruch 1 angegebene Bedeutung haben,
hydriert und die Verbindungen der Formel (V), gegebenenfalls nach ihrer Isolierung, mit etherspaltenden Mitteln in Verbindungen der Formel (I) überführt,

11. 2,2,3,3,4,4-Hexafluorcyclopentanol der Formel:

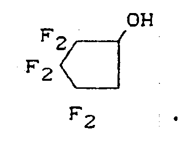

## Claims

1. Process for the preparation of partially fluorinated alcohols of the formula (I) 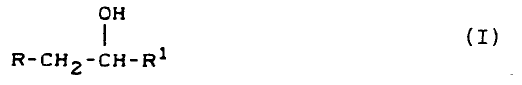 in which
R and R¹ are identical or different and represent alkyl which is monosubstituted or polysubstituted by fluorine, cycloalkyl which is monosubstituted or polysubstituted by fluorine or R and R¹ together represent the group -(CF₂)ₙ-, where n represents 2, 3 or 4,
characterized in that
a) compounds of the formula (II) 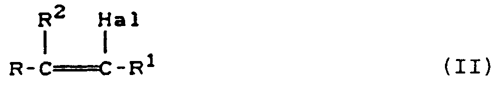 in which
R and R¹ have the abovementioned meaning,
R² represents hydrogen, fluorine, chlorine or bromine and
Hal represents halogen,
are reacted with an alcohol of the formula (III)
HO-R³ (III)
in which
R³ represents optionally substituted radicals from the series comprising alkyl, alkenyl, cycloalkyl, cycloalkenyl, aryl, heteroaryl and aralkyl,
in the presence of a base to give compounds of the formula (IV) 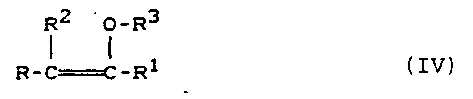 in which
R, R¹, R² and R³ have the abovementioned meaning,
and
b) the compounds of the formula (IV), if desired after their isolation, are hydrogenated catalytically with hydrogen,
either
α) in the absence of a base to give compounds of the formula (I),
or
β) in the presence of a base to give compounds of the formula (V) 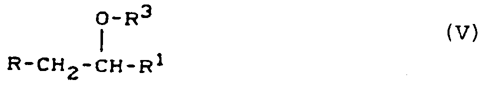 in which
R, R¹ and R³ have the abovementioned meaning,
and the compounds of the formula (V) are converted, if desired after their isolation, into compounds of the formula (I) using ether-cleaving agents.

2. Process according to Claim 1, characterized in that alkali metal or alkaline earth metal hydroxides, tertiary amines or alkoxides are employed as the base in step a.

3. Process according to Claim 1, characterized in that step a is carried out in the presence of diluents or an excess of the alcohol of the formula (III).

4. Process according to Claim 1, characterized in that step b is carried out in the presence of diluents.

5. Process according to Claim 1, characterized in that alkali metal and alkaline earth metal hydroxides, alkaline earth metal carbonates, tertiary organic amines or alkoxides are used in step b-β.

6. Process according to Claim 1, characterized in that the steps b-α and b-β (hydrogenation and ether cleavage) are carried out as a one-pot reaction.

7. Process according to Claim 1, characterized in that R and R¹ represent alkyl having 1 to 10 carbon atoms, which is monosubstituted or polysubstituted by fluorine, cycloalkyl having 3 to 10 carbon atoms, which is monosubstituted or polysubstituted by fluorine, or R and R¹ together represent the group -(CF₂)ₙ-, where n represents 2, 3 or 4.

8. Process according to Claim 1, characterized in that R³ represents in each case optionally substituted alkyl having 1 to 8 carbon atoms, alkenyl having 3 to 8 carbon atoms, cycloalkyl having 3 to 6 carbon atoms, cycloalkenyl having 4 to 10 carbon atoms, phenyl, naphthyl, pyridyl, pyrimidyl or phenylalkyl having 1 to 4 carbon atoms in the alkyl moiety.

9. Process according to Claim 8, characterized in that halogen or alkoxy having 1 to 4 carbon atoms and 1 or 2 oxo groups are selected as substituents.

10. Process for the preparation of the compounds of the formula (I) 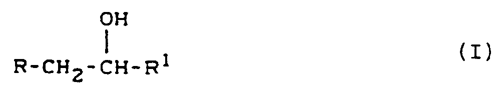 in which
R and R¹ have the meaning given in Claim 1,
characterized in that
the compounds of the formula (IV) 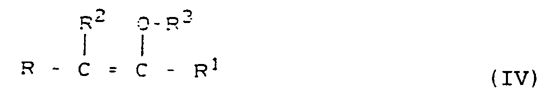 in which
R, R¹, R² and R³ have the meaning given in Claim 1, are hydrogenated catalytically with hydrogen,
either
α) in the absence of a base to give compounds of the formula (I)
or
β) in the presence of a base to give compounds of the formula (V) 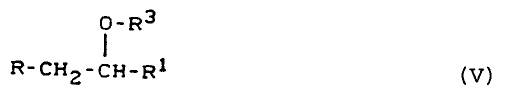 in which
R, R¹ and R³ have the meaning given in Claim 1,
and the compounds of the formula (V) are converted, if desired after their isolation, into compounds of the formula (I) using ether-cleaving agents.

11. 2,2,3,3,4,4-Hexafluorocyclopentanol of the formula:

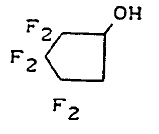

## Revendications

1. Procédé de production d'alcools partiellement fluorés de formule (I), 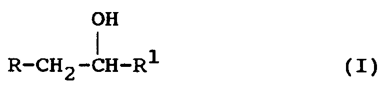 dans laquelle
R et R¹ sont identiques ou différents et représentent un groupe alkyle substitué une ou plusieurs fois par du fluor, un groupe cycloalkyle substitué une ou plusieurs fois par du fluor, ou bien R et R¹ forment conjointement le groupe -(CF₂)ₙ- dans lequel n a la valeur 2, 3 ou 4,
caractérisé en ce qu'on fait réagir :
a) des composés de formule (II), 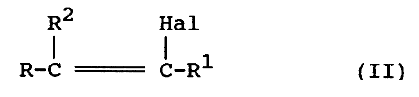 dans laquelle
R et R¹ ont la définition indiquée ci-dessus,
R² représente l'hydrogène, le fluor, le chlore ou le brome et
Hal est un halogène,
avec un alcool de formule (III),
HO-R³ (III)
dans laquelle
R³ représente des restes éventuellement substitués de la série alkyle, alcényle, cycloalkyle, cycloalcényle, aryle, hétéroaryle ou aralkyle,
en présence d'une base pour former des composés de formule (IV), 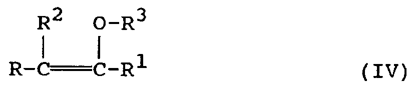 dans laquelle
R, R¹, R² et R³ ont la définition indiquée ci-dessus, et
b) on hydrogène les composés de formule (IV), éventuellement après les avoir isolés, par voie catalytique avec de l'hydrogène,
α) en l'absence d'une base pour former des composés de formule (I),
ou bien
β) en présence d'une base pour former des composés de formule (V) 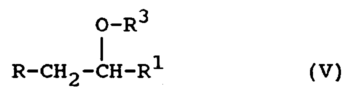 où
R R¹ et R³ ont la définition indiquée ci-dessus,
et on transforme les composés de formule (V), le cas échéant après les avoir isolés, en composés de formule (I) avec des agents qui coupent les éthers.

2. Procédé suivant la revendication 1, caractérisé en ce qu'on utilise comme base dans l'étape a des hydroxydes de métaux alcalins ou alcalino-terreux, des amines tertiaires ou des alcoolates.

3. Procédé suivant la revendication 1, caractérisé en ce qu'on conduit l'étape a en présence de diluants ou d'un excès de l'alcool de formule (III).

4. Procédé suivant la revendication 1, caractérisé en ce qu'on conduit l'étape b en présence de diluants.

5. Procédé suivant la revendication 1, caractérisé en ce qu'on utilise dans l'étape b-β des hydroxydes de métaux alcalins et alcalino-terreux, des carbonates de métaux alcalino-terreux, des amines organiques tertiaires ou des alcoolates.

6. Procédé suivant la revendication 1, caractérisé en ce qu'on conduit les étapes b-α et b-β (hydrogénation et coupure des éthers) comme réaction en récipient unique.

7. Procédé suivant la revendication 1, caractérisé en ce que R et R¹ représentent un groupe alkyle ayant 1 à 10 atomes de carbone substitué une ou plusieurs fois par du fluor, un groupe cycloalkyle ayant 3 à 10 atomes de carbone substitué une ou plusieurs fois par du fluor, ou bien R et R¹ forment conjointement le groupe -(CF₂)ₙ- dans lequel n a la valeur 2, 3 ou 4.

8. Procédé suivant la revendication 1, caractérisé en ce que R³ représente un groupe, éventuellement substitué, alkyle ayant 1 à 8 atomes de carbone, alcényle ayant 3 à 8 atomes de carbone, cycloalkyle ayant 3 à 6 atomes de carbone, cycloalcényle ayant 4 à 10 atomes de carbone, phényle, naphtyle, pyridyle, pyrimidyle ou phénylalkyle ayant 1 à 4 atomes de carbone dans la partie alkyle.

9. Procédé suivant la revendication 8, caractérisé en ce qu'on choisit comme substituants un halogène, un groupe alkoxy ayant 1 à 4 atomes de carbone ainsi que 1 ou 2 groupes oxo .

10. Procédé de production des composés de formule (I), 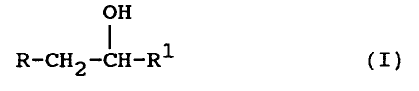 dans laquelle
R et R¹ ont la définition indiquée dans la revendication 1,
caractérisé en ce qu'on hydrogène les composés de formule (IV), 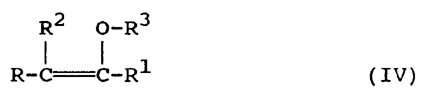 dans laquelle
R, R¹, R² R³ ont la définition indiquée dans la revendication 1, par voie catalytique avec de l'hydrogène,
α) en l'absence d'une base pour former des composés de formule (I),
ou bien
β) en présence d'une base pour former des composés de formule (V) 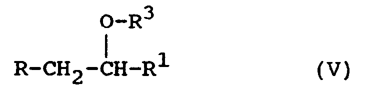
où
R R¹ et R³ ont la définition indiquée dans la revendication 1,
et on transforme les composés de formule (V), éventuellement après les avoir isolés, en composés de formule (I) avec des agents qui coupent les éthers.

11. Le 2,2,3,3,4,4-hexafluorocyclopentanol de formule
